(19) Europäisches Patentamt
European Patent Office
Office européen des brevets

(11) Veröffentlichungsnummer: **0 184 989**
**A1**

(12) **EUROPÄISCHE PATENTANMELDUNG**

(21) Anmeldenummer: **85810554.7**

(22) Anmeldetag: **20.11.85**

(51) Int. Cl.$^4$: **C 07 D 493/22**, A 61 K 31/35, A 01 N 43/24 // (C07D493/22, 313:00, 311:00, 311:00, 307:00)

(30) Priorität: **26.11.84 CH 5631/84**

(43) Veröffentlichungstag der Anmeldung: **18.06.86 Patentblatt 86/25**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Sturm, Elmar, Dr., Klusstrasse 66, CH-4147 Aesch (CH)**
Erfinder: **Maienfisch, Peter, Dr., Traugott Meyer-Strasse 5, CH-4147 Aesch (CH)**

(54) **5-Azolylacetoxi-Milbemycine als Ekto- und Endoparasitizide.**

(57) Milbemycin-Derivate der Formel I

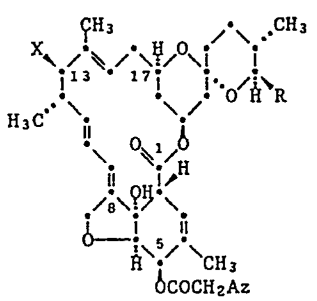

(I)

worin X Wasserstoff oder β-Halogen bedeutet, R Methyl, Ethyl, Isopropyl oder sek.-Butyl ist und Az einen in 1-Position verknüpften 5-gliedrigen heterocyclisch aromatischen Ring mit zwei bis vier Stickstoffatomen darstellt, der unsubstituiert oder ein- bis zweifach durch $C_{12}$-$C_6$ Alkyl substituiert ist, und ihre Säureadditionssalze und Metallkomplexverbindungen stellen aktive Präparate zur Bekämpfung von Endo- und Ektoparasiten dar, insbesondere zur Bekämpfung tierparasitären Nematoden. Sie lassen sich durch entsprechende Veresterung in 5-Position von Milbemycin-Derivaten gewinnen. Die gezielte 13-β-Halogenierung lässt sich aus 14,15-Epoxi-Milbemycin-Derivaten über die Zwischenstufe von $\Delta^{13,14}$-15-Hydroxi-Milbemycinen mit entsprechenden Halogenierungsmitteln erreichen.

EP 0 184 989 A1

 0184989

CIBA-GEIGY AG
Basel (Schweiz) 5-15169/=

## 5-Azolylacetoxi-Milbemycine als Ekto- und Endoparasitizide

Die vorliegende Erfindung betrifft 5-Azolylacetoxi-Milbemycin-Derivate der nachstehenden Formel I sowie ihre Salze und Metallkomplexverbindungen, ihre Herstellung und ihre Verwendung zur Bekämpfung von Schädlingen wie Ekto- und Endoparasiten, ferner Schädlingsbekämpfungsmittel, die mindestens eine dieser Verbindungen als Wirkstoff enthalten.

Die erfindungsgemässen Verbindungen haben die Formel I

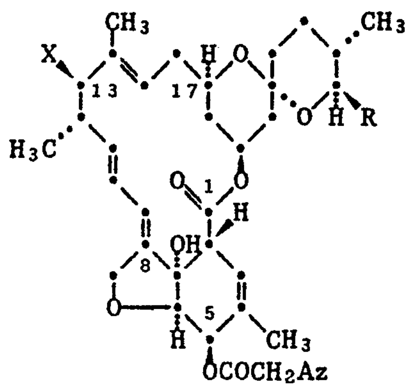

(I) .

In dieser Formel I bedeuten X Wasserstoff oder ß-Halogen, R Methyl, Ethyl, Isopropyl oder sek.Butyl und Az einen in 1-Position verknüpften 5-gliedrigen heterocyclisch aromatischen Ring mit 2-4 N-Atomen, der unsubstituiert oder ein- bis zweifach durch $C_1$-$C_6$Alkyl substituiert ist.

Beispiele für den letztgenannten Substituenten sind Imidazol, Pyrazol, 1,2,3-Triazol, 1,2,4-Triazol, 1,3,4-Triazol oder Tetrazol sowie ein- bis zweifach durch $C_1$-$C_6$-Alkylreste substituierte Azole wie 2-Ethyl-4-methylimidazol, 2-Isopropylimidazol, Methylimidazol, 3,5-Dimethyltriazol, Ethyltriazol, 3,4-Diethylpyrazol u.a.

Unter Halogen sind Fluor, Chlor, Brom oder Jod zu verstehen.

Salze werden aus Verbindungen der Formel I mit anorganischen oder organischen Säuren gebildet. Als Beispiele seien Chlorwasserstoffsäure, Bromwasserstoffsäure, Schwefelsäure, Salpetersäure, Phosphorsäure, Methansulfonsäure, p-Toluolsulfonsäure, Essigsäure, Oxalsäure, Weinsäure, Citronensäure, Ascorbinsäure, Sorbinsäure, Trimethylessigsäure, Benzoesäure, Salicylsäure, Bernsteinsäure, Maleinsäure genannt.

Als Metallkomplex-Bildner sind vor allem Kationen der I. und II. oder der IV. bis VIII. Nebengruppe des Periodensystems der Elemente geeignet. Beispiele sind Kupfer, Zink, Mangan, Chrom, Eisen, Nickel, Kobalt, Molybdän.

Die vorstehenden Aufzählungen stellen keine Limitierungen dar. Der Fachmann kennt weitere physiologisch verträgliche Salze und Komplexbildner.

Im Umfang der Formel I sind ganz klar diejenigen Milbemycin-Derivate bevorzugt, worin R für Isopropyl steht und X und Az die angegebenen Bedeutungen haben.

Wichtige Verbindungen sind jene der Formel I, worin X Wasserstoff oder Chlor, R Ethyl oder Isopropyl und "Az" 1,2,4-Triazol-1-yl bedeuten.

Eine besonders bevorzugte Verbindung ist 5-O-(1,2,4-Triazol-1'-yl)acetyl-13-β-chlor-milbemycin-D unter Einschluss seiner Salze und Metallkomplexe, insbesondere seiner Komplexe mit den Metallen Kupfer, Zink, Mangan, Chrom, Eisen, Nickel, Kobalt oder Molybdän.

Die Ausgangsmaterialien der Formel II, wobei X = Wasserstoff bedeutet, sind die bekannten Milbemycine.

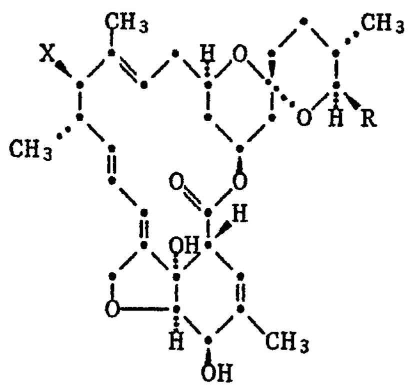

(II)

| | | |
|---|---|---|
| R = $CH_3$ | Milbemycin $A_3$ | aus US-PS 3,950,360 |
| R = $C_2H_5$ | Milbemycin $A_4$ | aus US-PS 3,950,360 |
| R = iso$C_3H_7$ | Milbemycin D | aus US-PS 4,346,171 |
| R = sec.$C_4H_9$ | 13-Desoxi-22,23-dihydro-C-076-Bla-aglycon, oder 13-Desoxi-22,23-dihydro-avermectin-Bla-aglykon aus US-PS 4,173,571. | |

Verbindungen, worin R sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13-Position) lassen sich jedoch gemäss US-PS 4,173,571 in Milbemycin-Homologe überführen.

Ausgangsverbindungen der Formel II, in denen R und X die vorstehend aufgeführten Bedeutungen haben, werden erfindungsgemäss in die Wirkstoffe I überführt, indem man nach Schema A

A:

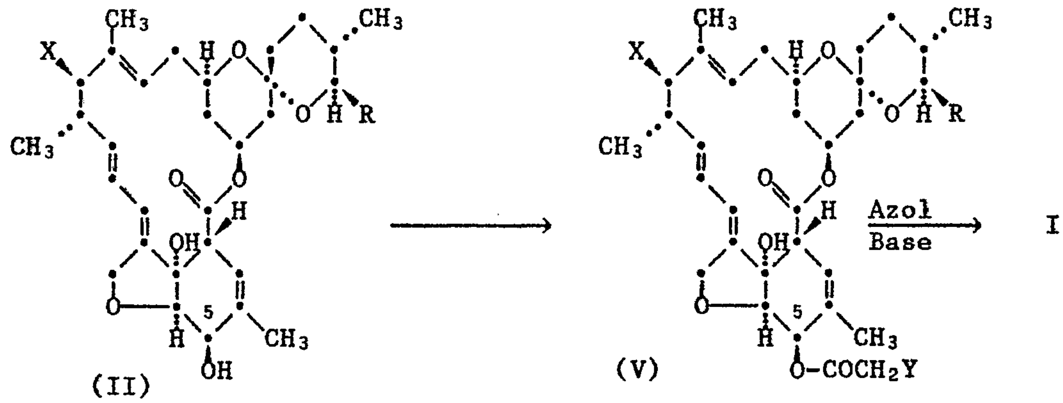


(II) (V) I

das Milbemycin-Derivat II an der 5-OH Gruppe mit einer substituierten Essigsäure

$$Y\text{-}CH_2\text{-}CO\text{-}Z$$

durch Veresterung in Verbindungen der Formel V überführt. Als Essigsäurederivate dienen zur Acetylierung befähigte Derivate wie entsprechend substituierte Acetylhalogenide oder Acetanhydride oder die substituierte Essigsäure selbst, die allesamt in 5-Position den Rest $Y\text{-}CH_2\text{-}CO\text{-}$ einführen. Z bedeutet also Halogen, bevorzugt Chlor oder Brom, -OH oder die halbe Sauerstoff-Funktion im Säureanhydrid. Y steht für Halogen, bevorzugt Chlor, Brom oder Jod für Azido oder für eine andere nucleophil leicht ersetzbare Abgangsgruppe wie beispielsweise Mesylat oder Tosylat.

Insbesondere seien hier genannt Chloracetylchlorid, Bromacetylbromid, Azidoacetylchlorid, Chloracetanhydrid, 2-Mesylessigsäurechlorid. Die Umsetzung erfolgt vorzugsweise in einem inerten, hydroxylgruppenfreien Lösungsmittel in Anwesenheit einer Base, wie Pyridin, Lutidin, Collidin, Trialkylamin, N-Dialkylanilin, oder bicyclische, nicht nucleophile Basen wie Diazabicyclooctan (DABCO) oder DBN.

Das solchermassen durch Y substituierte 5-O-Acetylderivat der Formel V wird nun mit einem Azol, bevorzugt in Gegenwart einer Base, zum Endprodukt I umgesetzt. Als Base dient entweder das Azol

selbst, indem man es im Ueberschuss einsetzt, oder ein tert.Amin wie Triethylamin, Pyridin, DABCO u.ä. Diese Umsetzungen verlaufen besonders glatt in aprotischen polaren Solventien wie DMSO, DMF, Dimethylacetamid u.a.

Nach Schema B können die Verbindungen der Formel I, einer abgekürzten erfindungsgemässen Verfahrensvariante folgend, hergestellt werden, indem man das Milbemycin-Derivat II direkt mit einer 2-Azolylessigsäure oder einem ihrer Säurederivate umsetzt:

B:

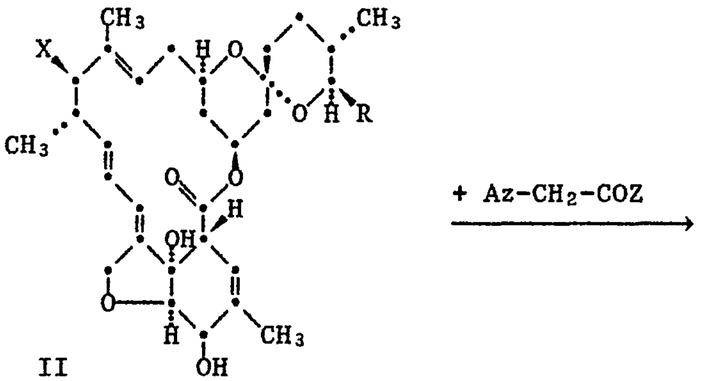


II

I

Hierin bedeutet Z Halogen, bevorzugt Chlor oder Brom, OH oder die halbe Sauerstoff-Funktion im Anhydrid $(Az-CH_2-CO)_2O$.

Im Falle der Säurehalogenide wird die Reaktion zweckmässig in Gegenwart einer tert.organischen wie oben genannten Base oder in Gegenwart einer anorganischen Base wie $NaHCO_3$ oder $K_2HPO_4$ oder $Mg(OH)_2$ durchgeführt. Die Veresterung mit Azolylacetanhydrid lässt sich ohne besondere Hilfsmittel durchführen. Vorteilhaft im Sinne einer Reaktionsbeschleunigung setzt man die Azolylessigsäure als solche ein und führt die Reaktion in Gegenwart wasserabspaltender Reagenzien durch. Beispielsweise arbeitet man in Anwesenheit von Dicyclohexylcarbodiimid und Pyridin oder in Gegenwart von Azodicarbonsäuredialkylester und Triphenylphosphin.

Beispiele für solche Essigsäurederivate sind

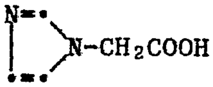 2-(Imidazol-1-yl)-essigsäure

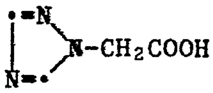 2-(1,2,4-Triazol-1-yl)-essigsäure

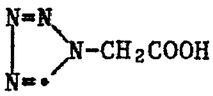 2-(1,2,3,4-Tetrazolyl)-essigsäure

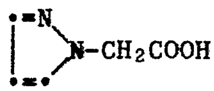 2-(Pyrazol-1-yl)-essigsäure

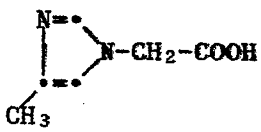 2-(4-Methylimidazol-1-yl)-essigsäure

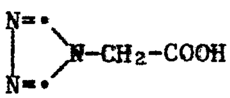 2-(1,2,4-Triazol-4-yl)-essigsäure

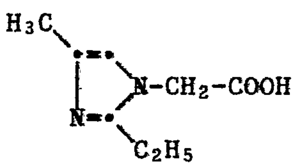 2-(2-Ethyl-4-methyl-imidazol-1-yl)-essigsäure

Diese beispielhaften Angaben stellen keine Limitierung dar.

Wie ersichtlich, stellt die weiter oben angeführte Reaktion A nur die Zerlegung der Reaktion B in zwei Teilschritte dar.

Ausgangsverbindungen der Formel II, in denen X ein beta-ständiges Halogenatom bedeutet, können aus Verbindungen der Formel II, worin X=H bedeutet, hergestellt werden. Hierzu wird das Epoxid III, das durch übliche Peroxid-Oxidation von Milbemycin-Derivaten der Formel II leicht erhältlich ist,

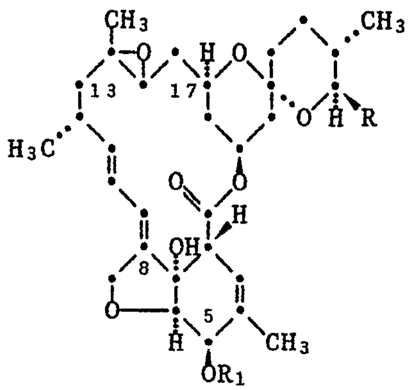

(III)

und worin $R_1$ ein Wasserstoffatom oder eine substituierte Silylschutzgruppe, wie z.B. Dimethyl-tert.butylsilyl, darstellt, vorerst mit Hilfe eines weiter unten beschriebenen Komplexreagenses $[HN_3]_m/[Al(Ethyl)_3]_n$ zum $\Delta^{13,14}$-Allylalkohol IV umgelagert:

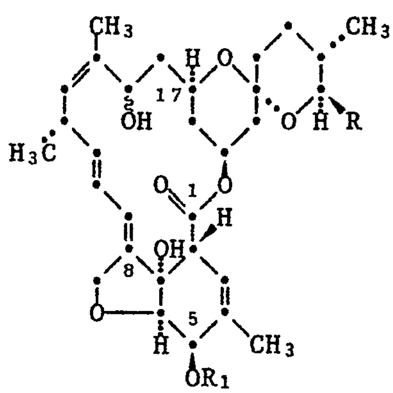

(IV)

Die Reaktion der Epoxidringöffnung wird vorteilhaft unter Schutzgas wie z.B. Stickstoff oder Argon durchgeführt.

Der Allylalkohol IV, in dem $R_1$ vorzugsweise für eine aliphatisch substituierte Silylgruppe steht, wird nun in charakteristischer und stereoselektiver Weise durch geeignete Halogenierungsmittel in das 13ß-Halogen-milbemycin-Derivat der Formel II (X = Halogen) überführt, wobei als gemeinsames Reaktionsprinzip eine Allyl-Umlagerung stattfindet. Beispielsweise dient zur 13ß-Fluorierung das Reagens Diethylaminoschwefeltrifluorid (DAST), zur 13ß-Chlorierung Thionylchlorid, zur 13ß-Bromierung Phosphortribromid und zur 13ß-Jodierung elementares Jod in Kombination mit Triphenylphosphin und Imidazol.

Die anschliessende Entfernung der Silylreste $R_1$ in der 5-Position geschieht durch selektive milde Hydrolyse ($\longrightarrow R_1$=H), die dem Fachmann geläufig ist, z.B. durch Verseifen mit einer aromatischen Sulfonsäure.

Das weiter oben genannte, zur Epoxid-Ringöffnung geeignete Komplex-Reagenz gehorcht der ungefähren Formel $[HN_3]_m/[Al(Ethyl)^3]_n$, worin m und n unabhängig voneinander die Zahl 1 oder 2 oder einen Zahlenwert zwischen 1 und 2 darstellen, und wird in inerten Lösungsmitteln aus den Einzelkomponenten $HN_3$ und $Al(C_2H_5)_3$ im Temperaturbereich von -30°C bis +10°C hergestellt. Es ist bei Minustemperaturen einige Zeit stabil. Es soll hier und im folgenden in vereinfachter Schreibweise als $HN_3/Et_3Al$ oder $HN_3/Al(C_2H_5)_3$ wiedergegeben werden. In Gegenwart des Komplexes reagiert eine beliebige Epoxi-Verbindung unter Oxiran-Ringöffnung und unter gleichzeitiger Bildung einer 1-Hydroxy-2-azido-Verbindung und eines substituierten Allylalkohols (=1-Hydroxi-$\Delta^{2,3}$-Verbindung).

Stickstoffwasserstoffsäure $HN_3$ lässt sich auch in statu nascendi in den $[HN_3]_m/[Al(Et)_3]_n$-Komplex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die Epoxidierung von Milbemycinen der Formel II mit X=H zu Verbindungen der Formel III wird in einer Lösungsmittelphase im Temperaturbereich von -10°C bis +20°C, vorzugsweise -5°C bis +5°C, durchgeführt.

Zur Epoxidierung kommen Persäuren wie Peressigsäure, Trifluorperessigsäure, Perbenzoesäure, Chlorperbenzoesäure und andere in Frage.

Die neuen als Zwischenprodukte verwendeten Ester der Formel V, worin R, und X die für die Formel I genannten Bedeutungen haben, sind ein weiterer Gegenstand vorliegender Erfindung. Ester der Formel V sind selbst wertvolle Wirkstoffe zur Bekämpfung von Schädlingen (z.B. Endoparasiten, Ektoparasiten und Insekten), wie nachfolgend ausführlicher beschrieben. Besonders hervorzuheben sind die 5-O-Chloracetylester und 5-O-Azidoacetylester der Milbemycin-Derivate der Formel V, die hervorragende anthelmintische und ektoparasitizide Eigenschaften besitzen.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und

Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### α-Herstellung von Zwischenprodukten der Formeln III und IV

### H-1. Herstellung von wasserfreiem $HN_3$

H-1.1. In Benzol. Die Herstellung erfolgte nach der Vorschrift von H. Wolf, Org. Reactions 3, 307 (1946). Die dabei erhaltene Lösung von $HN_3$ in Benzol wurde zweimal über wasserfreie, $Na_2SO_4$ (30 min. auf einer Flamme erhitzt und im Exsikkator abgekühlt) getrocknet und bei 0°C ⟶ 5°C durch Watte abfiltriert. Die Lösung wurde bei 4°C im Kühlschrank aufbewahrt.

H-1.2. In Ether. Die Herstellung erfolgte analog zur obigen Vorschrift, ausser dass bei der Zugabe der Schwefelsäure die Reaktionstemperatur zwischen -20°C und -10°C gehalten wurde; nach beendeter $H_2SO_4$ Zugabe wurde das Reaktionsgemisch auf -5°C erwärmt.

### H-2. Herstellung von 14,15-Epoxi-Milbemycin D (Formel III)

Zu einer Lösung von 550 mg Milbemycin D in 5 ml Dichlormethan wurde unter Eiskühlung eine Lösung von 170 mg Chlorperbenzoesäure in 5 ml Dichlormethan gegeben. Nach 1-stündigem Rühren bei 0° bis 5°C wurden nochmals 170 mg des Oxidationsmittels hinzugefügt und weitere 30 min. gerührt. Nach beendeter Reaktion wurde die Lösung in eine eisgekühlte Lösung von Natriumsulfit gegeossen und mit Essigsäureethylester extrahiert. Die vereinigten Extrakte wurden einmal mit Wasser gewaschen, getrocknet und im Vakuum eingedampft. Das Rohprodukt wurde durch Chromatographierung über eine Silicagel-Säule (Elutionsmittel: n-Hexan/Essigsäureethylester 20:15) gereinigt. Es wurden 450 mg 14,15-Epoxi-Milbemycin D als amorphe, weisse Substanz erhalten.

0184989

H-3. Herstellung von 15-Hydroxi-$\Delta^{13,14}$-Milbemycin D (Formel IV)

Es wurden bei -20°C zu einer Lösung von 2,1 ml (1,75 g, 15,3 mmol) Triethylaluminium in 8,5 ml (0,41 g, 9,53 mmol) einer 6,96 proz. Lösung von $HN_3$ in Diethyl-Ether gegeben, die dann bei -10°C unter stark exothermer Reaktion zu 1,8 g (3,15 mmol) 14,15-Epoxi-Milbemycin D (in Substanz) gegeben wurde. Nach 1 Stunde bei Raumtemperatur wurden 4 ml absoluter Ether zugegeben und das gallertige Reaktionsgemisch wurde kräftig gerührt. Nach 4 Stunden wurde wie in Vorschrift 2 aufgearbeitet und die Chromatographie an 70 g Kieselgel ($CH_2Cl_2$/Aceton 10:1) ergab 200 mg (10 %) 14-Azido-15-hydroxi-Milbemycin D und 820 mg (45 %) 15-Hydroxy-$\Delta^{13,14}$-Milbemycin D, Smp.: 151°C-153°C (aus Methanol).

H-4. Herstellung von 5-O-t-Butyldimethylsilyl-14,15-epoxi-Milbemycin D (Formel III)

Eine Lösung von 2,21 g (3,86 mmol) 14,15-Epoxi-Milbemycin D 757 mg (5,02 mmol) t-Butyldimethylchlorsilan und 342 mg (5,02 mmol) Imidazol in 4 ml DMF wurde 90 min. bei Raumtemperatur gerührt. Anschliessend wurden 80 ml Diethylether zugegeben, und das Gemisch wurde über 20 g Kieselgel filtriert und eingeengt. Es wurden 2,65 g (100 %) 5-O-t-Butyldimethylsilyl-14,15-epoxi-Milbemycin D erhalten.

$^1$H-NMR (300 MHz. Lösungsmittel $CDCl_3$. Messwerte $\delta$ bezogen auf $Si(CH_3)_4$ = TMS).

0,12 ppm (s) $(CH_3)_2Si-O-$;

0,92 ppm (s) $(t.-C_4H_9)Si-O-$;

1,23 ppm (breites s) ($C_{14}CH_3$, d.h. Signal der $CH_3$-Gruppe in 14-Position);

2,56 ppm (d; J = 9) ($C_{15}H$, d.h. Signal des Protons in 15-Position).

Ahnlich lässt sich durch Reaktion mit Trimethylsilyl-trifluormethansulfonat das entsprechende 5-O-Trimethylsilyl-14,15-epoxi-Milbemycin D herstellen, Smp. 92-97°C.

H-5. Herstellung von 5-O-t-Butyldimethylsilyl-15-hydroxi-$\Delta^{13,14}$-Milbemycin D (Formel IV)

Eine Lösung des $HN_3/Et_3Al$ - Komplex-Reagenz (hergestellt aus einer Lösung von 4,97 ml Triethylaluminium in 7 ml abs. Tetrahydrofuran (THF) und 9,15 ml einer 2,39 M Lösung von $NH_3$ (21,9 mmol) in abs. Diethylether wurde unter Argon zu einer Lösung von 5.0 g (7,29 mmol) 5-O-Butyldimethylsilyl-14,15-epoxi-Milbemycin D in ca. 20 ml abs. THF gegeben, und das Gemisch wurde 16 Std. unter Rückfluss erhitzt. Anschliessend wurden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wurde filtriert, eingeengt , und die Chromatographie des Rohproduktes an 160 g Kieselgel (0 - 30 % Essigsäureethylester in Hexan) ergab 2,37 g (47 %) 5-O-t-Butyldimethylsilyl-15-hydroxi-$\Delta^{13,14}$-Milbemycin D.

$^1$H-NMR (300 MHz, $CDCl_3$):
1,59 (d; J = 1) ($C_{14}CH_3$);
4,06 (dd; $J_1$ = 11;$J_2$ = 4) ($C_{15}H$);
5,15 (d; J = 8) ($C_{13}H$).

Daneben wurden 109 mg (2 %) 13β-Azido-5-O-t-butyldimethylsilyl-Milbemycin D gewonnen.

H-6. Herstellung von 14,15-Epoxi-Milbemycin $A_4$ (Formel III) ($R_2$=$C_2H_5$)

Zu einer Lösung von 5,7 g (10,5 mmol) Milbemycin $A_4$ in 140 ml Dichlormethan und 120 ml 0,5 M $NaHCO_3$-Lösung wurde bei Raumtemperatur eine Lösung von 2,43 g (14,08 mmol) m-Chlorobenzopersäure in 760 ml Dichlormethan tropfenweise zugegeben. Das Gemisch wurde 1 Std. bei Raumtemperatur kräftig gerührt und dann mit 300 ml Dichlormethan verdünnt. Die organische Phase wurde mit wässriger $NaHCO_3$-Lösung gewaschen, mit $Na_2SO_4$ getrocknet und eingeengt. Es wurden 5,7 g Epoxid als Rohprodukt erhalten.

H-7. Herstellung von 5-O-t-Butyldimethylsilyl-14,15-epoxi-Milbemycin $A_4$ (Formel III)

5,7 g 14,15-Epoxi-Milbemycin $A_4$ wurden in 10 ml trockenem Dimethylformamid (DMF) gelöst. Bei Raumtemperatur wurden 0,63 g (9,16 mmol) Imidazol und 1,4 g (9,34 mmol) t-Butyldimethylchlorsilan zugegeben. Das Gemisch wurde 1 Std. bei Raumtemperatur gerührt und an 150 g Kieselgel chromatographiert (Hexan/Ether 4:1),wobei 2,84 g (40 % d. Th. Ausbeute, bezogen auf Milbemycin $A_4$) des silylierten Epoxi-Derivates erhalten wurden.

H-8. Herstellung von 5-O-t-Butyldimethylsilyl-15-hydroxi-$\Delta^{13,14}$-Milbemycin $A_4$ (Formel IV)

Das Komplex-Reagenz $HN_3$/Al (Ethyl)$_3$ wurde wie folgt hergestellt: 2,8 ml (12,2 mmol) $Al(C_2H_5)_3$ in 4 ml abs. THF wurden unter Argon bei ca. -20°C langsam mit 5,28 ml (20,4 mmol) einer 10%igen Lösung von $HN_3$ in abs. Diethylether versetzt. Zu dieser Lösung wurde unter Argon eine Lösung von 2,84 g (4,25 mmol) der im Beispiel 7 erhaltenen Verbindung gegeben, und das so erhaltene Gemisch wurde 4 Std. unter Rückfluss erhitzt. Bei Raumtemperatur wurden 500 ml Diethyl-Ether, 10 g $Na_2SO_4 \cdot 10H_2O$ und 10 g Celite zugegeben, und das Gemisch wurde filtriert und eingeengt. Die Chromatographie des Rohproduktes an 100 g Kieselgel (Hexan/Diethylether 7:2) ergab 1,72 g (60 % d.Th.) der Titel-Verbindung.

$^1$H-NMR (300 MHz, $CDCl_3$):
1,59 (br. s) ($C_{14}CH_3$);
4,05 (br. s) ($C_{15}H$);
5,15 (d; J = 6) ($C_{13}H$).

Daneben wurden 0,1 g 13β-Azido-5-O-t-butyldimethylsilyl-Milbemycin $A_4$ erhalten.

H-9. Hestellung von 15-Hydroxi-$\Delta^{13,14}$-Milbemycin $A_4$ (Formel IV)

Die Hydrolyse der im Beispiel 8 genannten Titelverbindung mit einer 1%igen Lösung von p-Toluolsulfonsäure in Methanol und die Aufarbeitung in Diethylether mit 5%ig. Na-Hydrogencarbonat-Lösung ergab die Titel-Verbindung.

H-10. Herstellung von 14,15-Epoxi-Milbemycin $A_3$ (Formel III) ($R_2$ = $CH_3$)

Nach der Vorschrift des Beispiels H-2 werden aus 220 mg Milbemycin $A_3$ in 5 ml Dichlormethan und 320 mg Benzopersäure in 5 ml Dichlormethan bei -2° bis +5°C während 1,5 Std. und Reinigung über eine Silicagel-Säule 190 mg 14,15-Epoxid-Milbemycin $A_3$ gewonnen.

H-11. Herstellung von 5-O-Methyldiphenylsilyl-14,15-epoxi-Milbemycin $A_3$ (Formel III)

Nach der Vorschrift des Beispiels H-4 werden aus 190 mg 14,15-Epoxi-Milbemycin $A_3$ und 120 mg Diphenylmethylchlorsilan in Gegenwart von Imidazol 217 mg der Titel-Verbindung erhalten.

H-12. Herstellung von 5-O-Methyldiphenylsilyl-15-hydroxi-$\Delta^{13,14}$-Milbemycin $A_3$ (Formel IV)

Analog zur Epoxid-Spaltung des Beispiels H-5 werden aus 210 mg 5-O-Methyldiphenylsilyl-14,15-epoxi-Milbemycin $A_3$ in absolutem Diethylether mit Hilfe des Komplex-Reagenz $HN_3/Et_3Al$ unter Argon nach anschliessender Reinigung 203 mg der Titelverbindung erhalten.

$^1$H-NMR (300 HMz $CDCl_3$);
1,58 (br. s) ($C_{14}CH_3$);
4,05 (br. s) ($C_{15}H$);
5,15 (d; J=6)($C_{13}H$).

H-13. Herstellung von 15-Hydroxi-$\Delta^{13,14}$-Milbemycin $A_3$ (Formel IV)

Analog zu Beispiel H-2 wird das Reagenz $HN_3/Al(C_2H_5)_3$ frisch hergestellt und bei -10°C zu einer Lösung von 830 mg (3,05 mmol) 14,15-Epoxi-Milbemycin $A_3$ in 7 ml trockenem Diethylether getropft. Nach der Aufarbeitung werden 385 mg 15-Hydroxi-$\Delta^{13,14}$-Milbemycin $A_3$ und 92 mg 4-Azido-15-hydroxi-Milbemycin $A_3$ erhalten.

H-14. Herstellung von 13-Deoxi-14,15-epoxi-22,23-dihydro-avermectin-Bla-aglykon ($R_2$ = sec.$C_4H_9$) (Formel III)

Analog zu Beispiel H-6 erhält man aus 520 mg 13-Deoxi-22,23-Dihydro-avermectin-Bla-aglykon [Tetrahedron Letters, Vol. 24, No. 48, pp. 5333-5336 (1983)] und 210 mg m-Chlorbenzopersäure in 20 ml Dichlormethan 510 mg der Titelverbindung.

H-15. Herstellung von 5-O-t.-Butyldimethylsilyl-13-deoxi-14,15-epoxi-22,23-dihydro-avermectin-Bla-aglykon (Formel III)

Analog zu Beispiel H-7 erhält man aus 220 mg der Titelverbindung von Beispiel 14 und 55 mg tert.-Butyldimethylchlorsilan in Gegenwart von 25 mg Imidazol in 5 ml trockenem DMF 108 mg der Titelverbindung.

H-16. Herstellung von 13-Deoxi-15-hydroxy-$\Delta^{13,14}$-22,23-dihydro-avermectin-Bla-aglykon (Formel IV)

Analog zu Beispiel H-3 erhält man aus 220 mg der Titelverbindung von Beispiel 15 mit dem Komplex-Reagenz, bestehend aus 320 mg $Al(C_2)H_5)_3$ und 110 mg einer 6,96%igen Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykon erhalten.

β-Herstellung von Verbindungen der Formel II (X = Halogen)

H-17. Herstellung von 5-O-tert.Butyldimethylsilyl-13β-fluoro-milbemycin D und von 13β-Fluoro-Milbemycin D

Eine Lösung von 18,3 µ (24 mg; 0,15 mmol) Diethylaminoschwefeltrifluorid (DAST) in 0,15 ml trockenem Dichlormethan wurde bei -60°C unter Argon zu einer Lösung von 103 mg (0,15 mmol) 5-O-tert.Butyl-dimethylsilyl-15-hydroxy-$\Delta^{13,14}$-Milbemycin D in 1,5 ml Dichlormethan getropft. Nach 10 min. wurde mit 5%iger wässriger $NaHCO_3$-Lösung und Diethylether aufgearbeitet. Die organische Phase wurde mit $MgSO_4$ getrocknet und eingeengt. Man erhielt 100 mg 5-O-tert.Butyldimethyl-silyl-13β-fluoro-milbemycin D, das mit 2 ml einer 1%igen Lösung von p-Toluolsulfonsäure in Methanol 10 Std. bei Raumtemperatur gerührt und dann mit 5%iger wässriger $NaHCO_3$-Lösung und durch Ausschütteln mit dreimal 2 ml Diethylether aufgearbeitet wurde. Die Chromatographie des Rohprodukts an 20 g Kieselgel (Laufmittel Aceton/Dichlormethan 1:12) ergab 58 mg (67 % d.Th.) 13β-Fluoro-Milbemycin D.

$^1$H-NMR (300 MHz; $CDCl_3$; TMS):
1,61 ppm (br. s)($C_{14}CH_3$)
2,5-2,7 ppm (m)($C_{12}H$)
4,40 ppm (dd; $J_1$=48; $J_2$=10)($C_{13}H$).

Massenspektrum m/e: 574 ($M^+$, 6 % von $C_{33}H_{47}FO_7$), 446, 374, 332, 428, 151.

H-18. Herstellung von 5-O-tert.Butyldimethylsilyl-13β-fluoro-milbemycin $A_4$ und von 13β-Fluoromilbemycin $A_4$.

Analog zu Beispiel H-17 erhält man aus 202 mg 5-O-tert.Butyldi-methylsilyl-15-hydroxy-$\Delta^{13,14}$-milbemycin $A_4$ und 36,2 µl DAST 183 mg 5-O-tert.Butyldimethylsilyl-13β-fluoromilbemycin $A_4$ und durch Entsilylierung mit methanolischer p-Toluolsulfonsäure 114 mg 13β-Fluromilbemycin $A_4$ .

$^1$H-NMR (300 MHz; $CDCl_3$; TMS):
1,61 (s)($C_{14}CH_3$)
4,42 (dd; $J_1$=47; $J_2$=10)($C_{13}H$).

H-19. Herstellung von 5-O-Methyldiphenylsilyl-13β-fluoro-milbemycin $A_3$ und von 13-β-Fluoro-milbemycin $A_3$.

Analog zu Beispiel H-17 erhält man aus 108 mg 5-O-Methyldiphenyl-silyl-15-hydroxi-$\Delta^{13,14}$-Milbemycin $A_3$ und 19,0 µl DAST 81 mg 5-O-Methyldiphenylsilyl-13β-fluoro-milbemycin $A_3$ und durch Entsilylierung mit methanolischer p-Toluolsulfonsäure in 5-Position 68 mg 13β-Fluoro-milbemycin $A_3$ .

H-20. Herstellung von 5-O-tert.Butyldimethylsilyl-13β-chlor-milbemycin D und von 13β-Chlor-milbemycin D

Zu einer Lösung von 50 mg (0,073 mmol) 5-O-tert.Butyldimethylsilyl-15-hydroxi-$\Delta^{13,14}$-Milbemycin D in 1,5 ml trockenem Dichlormethan wurden unter Argon und bei Eis/Methanol-Kühlung (ca. -10°C) 8,5 µl (13,9 mg, 0,116 mmol) Thionylchlorid getropft. Die Reaktion wird begünstist durch die Gegenwart von 2 Aequivalenten (0,146 mmol) Triethylamin. Nach 5 min. wurde mit 5%iger wässriger $NaHCO_3$-Lösung und dann mit Diethylether ausgeschüttelt. Die organische Phase wurde mit $MgSO_4$ getrocknet und eingeengt. Die Säulenchromatographie des Rohprodukts an 20 g Kieselgel (Laufmittel Ethylacetat/Hexan 1:20) lieferte 17 mg (30 % d.Th.) 5-O-tert.Butyldimethylsilyl-13β-chlor-milbemycin D.

$^1$H-NMR (300 MHz; $CDCl_3$; TMS):
0,93 (s)($C_5$-OSiC$(CH_3)_3$-)
0,13 (s)($C_5$-OSi$(CH_3)_2$-)
2,45-2,65 (m)($C_{12}H$)
4,08 (d; J=11)($C_{13}H$).

Die Behandlung der silylierten Verbindung mit 1%iger p-Toluolsulfonsäure in Methanol während 1 Stunde lieferte nach Behandeln mit 5%iger wässriger $NaHCO_3$-Lösung, Aufnahme in Diethylether und Chromatographie an Kieselgel (Laufmittel Ethylacetat/Hexan 2:3) in quantitativer Ausbeute 13β-Chlormilbemycin-D.

$^1H$-NMR (300 MHz; $CDCl_3$; TMS):
1,67 (s)($C_{14}CH_3$);
5,24 (d; J=11)($C_{13}$ αH).

H-21. Herstellung von 5-O-tert.Butyldimethylsilyl-13β-brom-milbemycin D und von 13β-Brom-milbemycin D

Zu einer Lösung von 182 mg (0,265 mmol) 5-O-tert.Butyldimethylsilyl-15-hydroxi-$\Delta^{13,14}$-milbemycin D in 5 ml trockenem Dichlormethan wurden unter Argon bei -10°C 0,013 ml (36 mg, 0,133 mmol) $PBr_3$ unter Rühren zugetropft. Nach 5 min. wurde 0,1 ml Methanol zugegeben, mit Ether verdünnt, und die organische Phase wurde mit einer 5%igen wässrigen $NaHCO_3$-Lösung gewaschen, mit $MgSO_4$ getrocknet und durch Kieselgel filtriert. Die Chromatographie des Rohproduktes an 20 g Kieselgel (Ethylacetat/Hexan 1:4 [100 ml] und 2:3 [250 ml]) ergab 98 mg (49 % d.Th.) 5-O-tert.Butyldimethylsilyl-13β-brom-milbemycin D und 50 mg (30 % d.Th.) 13β-Brom-milbemycin D.
Die Behandlung des t-Butyldimethylsilyläther-Derivats mit 2 ml einer 1%igen Lösung von p-Toluolsulfonsäure in Methanol (1 Std. bei Raumtemperatur) gefolgt von der Aufarbeitung in Diethylether mit 5%iger wässriger $NaHCO_3$-Lösung und der Chromatographie an Kieselgel (Ethylacetat/Hexan 2:3) ergab in quantitativer Ausbeute 13β-Brom-Milbemycin D.

$^1H$NMR (300 MHz; $CDCl_3$; TMS):
1,68 (s)($C_{14}CH_3$);
1,87 (s)($C_4CH_3$);
4,30 (d; J=10,7)($C_{13}$H).

Massenspektrum m/e: 636, 634 ($M^+$; $C_{33}H_{47}BrO_7$), 508, 506, 427, 209, 149, 41.

H-22. Herstellung von 5-O-tert.Butyldimethylsilyl-13β-jod-milbemycin D und von 13β-Jod-milbemycin D

Eine Lösung von 526 mg (0,776 mmol) 5-O-tert.Butyldimethylsilyl-15-hydroxi-$\Delta^{13,14}$-milbemycin D, 493 mg (1,884 mmol) Triphenylphosphin, 128 mg (1,884 mmol) Imidazol und 292 mg (1,149 mmol) Jod in 10 ml trockenem Dichlormethan wurde 20 min. bei Raumtemperatur unter Argon gerührt. Das Gemisch wurde mit Diethylether vedünnt, mit 5%iger wässriger $NaHCO_3$-Lösung gewaschen, und die organische Phase wurde mit $MgSO_4$ getrocknet. Die Chromatographie des Rohproduktes an 75 g Kieselgel (Ethylacetat/Hexan 1:12 [500 ml]) ergab 390 mg (64 %) 5-O-tert.Butyldimethylsilyl-13β-jod-milbemycin D.

159 mg (0,20 mmol) dieser silylierter Verbindung wurden mit 2 ml einer 1%igen Lösung von p-Toluolsulfonsäure in Methanol 1 Std. lang bei Raumtemperatur aufgearbeitet. Die Säulenchromatographie an 20 g Kieselgel (Laufmittel Ethylacetat/Hexan 2:3) ergab 103 mg (76 % d.Th.) 13β-Jod-milbemycin D).

$_1$H-NMR (300 MHz; $CDCl_3$; TMS):
1,64 (s)($C_{14}CH_3$)
1,82 (s)($C_4CH_3$
4,52 (d; J=11,0)($C_{13}H$).

H-23. Herstellung von 5-O-tert.Butyldimethylsilyl-13β-chloro-milbemycin $A_4$ und von 13β-Chloro-milbemycin $A_4$.

Zu einer Lösung von 198 mg (0,295 mmol) 5-O-tert.Butyldimethylsilyl-15-hydroxi-$\Delta^{13,14}$-Milbemycin $A_4$ und 0,082 ml (=60 mg; 0,589 mmol) Triethylamin in 5 ml trockenem Dichlormethan wurden unter Argon bei -10°C unter Rühren 0,026 ml (=42 mg; 0,354 mmol) Thionylchlorid getropft. Nach 5 min. wurden 0,1 ml Methanol zugegeben. Das Gemisch wurde mit 5%iger wässriger $NaHCO_3$-Lösung und Diethylether (=Ether)

aufgearbeitet. Die Chromatographie an 20 g Kieselgel (Ethylacetat/Hexan 1:19) ergab 96 mg (47 %) 5-O-tert.Butyldimethylsilyl-13β-chloro-milbemycin $A_4$ .

Diese Produkt wurde mit 2 ml einer 1%igen Lösung von p-Toluolsulfonsäure in Methanol 1 Std. lang bei Raumtemperatur gerührt, mit 5%iger wässriger $NaHCO_3$-Lösung behandelt und an 20 g Kieselgel (Ethylacetat/Hexan 2:3) chromatographiert. Man erhielt 66 mg (82 % d.Th.) 13β-Chlor-Milbemycin $A_4$.

$^1$H-NMR (250 MHz; $CDCl_3$; TMS):
1,67 (s)($C_{14}CH_3$)
3,10 (t)(J=ca. 7Hz)($C_{25}H$)
5,24 (d)(J=ca. 11Hz)($C_{13}H$)
Massenspektrum m/e: 576 ($M^+$, $C_{32}H_{45}O_7Cl$)
448, 442, 348, 279, 195, 167, 151.

H-24. Herstellung von 5-O-Methyldiphenylsilyl-13β-chloro-milbemycin $A_3$ und von 13β-Chloromilbemycin $A_3$.

Nach der Vorschrift des Beispiels H-23 werden aus 194 mg 5-O-Methyldiphenylsilyl-15-hydroxi-$\Delta^{13,14}$-Milbemycin $A_3$ und 0,0025 ml $SOCl_2$ 122 mg 5-O-Methyldiphenylsilyl-13β-chloro-milbemycin $A_3$ und nach Abspaltung des Silylrests 83 mg 13β-Chloro-milbemycin $A_3$ erhalten.

$^1$H-NMR (250 MHz; $CDCl_3$; TMS):
1,66 (s) ($C_{14}CH_3$)
1,90 (s) ($C_4CH_3$)
5,23 (d) (J=11 Hz) ($C_{13}H$).

H-25. Herstellung von 5-O-tert.Butyldimethylsilyl-13α-chloro-22,23-dihydroavermectin-Bla-aglykon und von 13α-chloro-22,23-dihydroavermectin-Bla-aglykon

Zu einer Lösung von 327 mg (0,467 mmol) 5-O-tert.Butyldimethylsilyl-22,23-dihydroavermectin-Bla-aglykon und 0,130 ml (=95 mg; 0,934 mmol) Triethylamin in 10 ml trockenem Dichlormethan wurden unter Argon bei -10°C 0,041 ml (=67 mg; 0,561 mmol) $SOCl_2$ unter Rühren getropft. Nach 5 min. wurden 0,1 ml Methanol zugegeben. Das Gemisch wurde mit 5%iger wässriger Na-Hydrogencarbonat-Lösung und Ether aufgearbeitet. Die Chromatographie des Rohprodukts an 20 g Kieselgel (Ethylacetat/Hexan 1:9) ergab 117 mg (50 % d.Th.) 5-O-tert.Butyldimethylsilyl-13α-chloro-22,23-dihydroavermectin-Bla-aglykon.

Dieses Produkt wurde mit 2 ml einer 1%igen Lösung von p-Toluolsulfonsäure in $CH_3OH$ während 1 Std. Rühren bei Raumtemperatur entsilyiert, dann mit 5%iger wässariger Na-Hydrogencarbonat-Lösung und Ether aufgearbeitet und an 20 g Kieselgel chromatographiert (Ethylacetat/Hexan 2:3). Man erhielt 68 mg (69 % d.Th.) 13β-Chloro-22,23-dihydroavermectin-Bla-aglykon.

$^1$H-NMR (300 MHz; $CDCl_3$; T>MS):
1,63 (s) ($C_{14}CH_3$)
1,87 (s) ($C_4CH_3$)
4,39 (s) ($W_{1/2}$=6 Hz)($C_{13}H$).

Massenspektrum m/e: 604 ($M^+$, $C_{34}H_{49}O_7Cl$), 476, 348, 223, 195, 151.

γ-Herstellung von Verbindungen der Formel V.

H-26. Herstellung von 5-O-Chloracetyl-milbemycin D. (=Z-1)
Zu einer Lösung von 400 mg Milbemycin D in 10 ml Pyridin wurden unter Eiskühlung (0-5°C) 168 mg Chloracetylchlorid mittels einer Injektionsspritze langsam zugetropft. Die gelblichtrübe Reaktionslösung wurde nach 2 Stunden Rühren bei 0°C auf 100 ml eiskalte 1 N wässrige Chlorwasserstoffsäure gegossen und viermal mit je 20 ml Diethylether (=Ether) extrahiert. Die sorgfältig gewaschene und getrocknete Etherlösung ergab nach dem Eindampfen im Vakuum ein amorphes, schaumiges Produkt, das durch Säulenchromatographie an Silicagel mit dem Laufmittel Methylenchlorid/Methanol (98:2) gereinigt wurde. Man erhielt 410 mg des Endprodukts als farblose, amorphe Masse.

$^1$H-NMR (300 MHz; CDCl; TMS = Tetramethylsilan) 5,32 (s) ($-CH_2-Cl$), 3,25 (schmales Multiplett) ($C_2H$; d.h. H-Signal in Position 2); Massenspektrum m/e: 632 ($M^+$; $C_{35}H_{49}CLO_8$).

H-27. Herstellung von 5-O-Bromacetyl-milbemycin $A_4$ (=Z-2)

Analog zur Vorschrift H-26 erhielt man aus Milbemycin $A_4$ ($R=C_2H_5$) und Bromacetylbromid in Pyridin 5-O-Bromacetylmilbemycin $A_4$ als amorphes Festprodukt.

H-28. Herstellung von 5-O-Jodacetyl-milbemycin D (=Z-3)
150 mg 5-O-Chloracetyl-milbemycin D und 50 mg Kaliumjodid wurden in 10 ml Aceton gelöst und 24 Stunden bei Raumtemperatur gerührt. Nach dem Verdünnen der Reaktionslösung mit der 5-fachen Menge Wasser wurde viermal mit je 10 ml Diethylether extrahiert und die aus der Etherlösung gewonnene Rohsubstanz an Silicagel chromatographiert (siehe H-26). Man erhielt 80 mg Reinprodukt. Smp. 133-137°C.

H-29. Herstellung von 5-O-Azidoacetyl-milbemycin D (=Z-4)
Analog zur Vorschrift H-28 erhielt man aus 150 mg 5-O-Chloracetyl-milbemycin D und 35 mg Natriumazid ($NaN_3$) 92 mg 5-O-Azidoacetyl-milbemycin D.

H-30. Herstellung von 5-O-Chloracetyl-13β-chlor-milbemycin $A_4$ (=Z-10)

Zu einer Lösung von 650 mg 13β-Chlor-milbemycin $A_4$ in 6 ml Methylenchlorid wurden 0,90 ml Pyridin und dann bei 0°C innerhalb 30 Min. 0,14 ml Chloracetylchlorid zugegeben. Nach 2 Stunden Rühren bei 0°C wurde das Reaktionsgemisch auf 100 ml 0,2 N HCl-Lösung gegossen und dreimal mit je 50 ml Diethylether extrahiert. Die organischen Phasen wurden vereinigt, dann mit 100 ml ges. NaCl-Lösung gewaschen, über $MgSO_4$ getrocknet, filtriert und eingeengt. Das erhaltene Rohprodukt ergab nach Chromatographie an Kieselgel mit Hexan/Diethylether 2:1 524 mg 5-O-Chloracetyl-13β-chlor-milbemycin $A_4$.

$^1$H-NMR (250 MHz, $CDCl_3$):
3,06 (dt, $J_d$=3, $J_t$=9) ($C_{25}H$)
4,14 (d, J=13) ($C_{13}H$)
4,17 (s) ($CH_2Cl$)

Nach Art der vorstehenden Beispiele lassen sich auch folgende Zwischenpodukte der Formel V gemäss vorliegender Erfindung herstellen:

| Nr. | |
|---|---|
| Z5. | 5-O-Bromacetyl-milbemycin $A_4$ |
| Z6. | 5-O-Bromacetyl-milbemycin-D |
| Z7. | 5-O-Chloracetyl-milbemycin $A_4$ |
| Z8. | 5-O-Chloracetyl-13ß-chlormilbemycin D |
| Z9. | 5-O-Chloracetyl-13ß-fluormilbemycin D |
| Z10. | 5-O-Chloracetyl-13ß-chlormilbemycin $A_4$ |
| Z11. | 5-O-Chloracetyl-13ß-chlormilbemycin $A_3$ |
| Z12. | 5-O-Jodacetyl-13ß-chlormilbemycin D |
| Z13. | 5-O-Chloracetyl-13ß-brommilbemycin $A_4$ |
| Z14. | 5-O-Fluoracetyl-milbemycin D |
| Z15. | 5-O-Methylsulfonyloxy-acetyl-milbemycin D |
| Z16. | 5-O-p-Tosyloxy-acetyl-milbemycin D |
| Z17. | 5-O-Azidoacetyl-milbemycin $A_3$ |

Z18. 5-O-Azidoacetyl-milbemycin $A_4$

Z19. 5-O-Ethylsulfonyloxy-acetyl-milbemycin $A_4$

Z20. 5-O-Chloracetyl-13-desoxy-22,23-dihydro-avermectin-Bla-aglykon

Z21. 5-O-Bromacetyl-13-desoxy-22,23-dihydro-avermectin-Bla-aglykon

Z22. 5-O-Azidoacetyl-13-desoxy-22,23-dihydro-avermectin-Bla-aglykon

Z23. 5-O-Chloracetyl-13-desoxy-13ß-chlor-22,23-dihydro-avermectin-Bla-aglykon

Z24. 5-O-Bromacetyl-13-desoxy-13ß-chlor-22,23-dihydro-avermectin-Bla-aglykon

Z25. 5-O-Bromacetyl-13ß-chlormilbemycin D

Z26. 5-O-Bromacetyl-13ß-chlormilbemycin $A_4$

Z27. 5-O-Bromacetyl-13ß-chlormilbemycin $A_3$

Z28. 5-O-Fluoracetyl-13ß-chlormilbemycin $A_4$

Z29. 5-O-Fluoracetyl-13ß-Chlormilbemycin D

Z30. 5-O-Azidoacetyl-13ß-chlormilbemycin $A_4$

Z31. 5-O-Azidoacetyl-13ß-chlormilbemycin D

Z32. 5-O-Azidoacetyl-13-desoxy-13ß-chlor-22,23-dihydro-avermectin-Bla-aglykon

Z33. 5-O-Benzolsulfonyloxyacetyl-13ß-chlormilbemycin $A_4$

Z34. 5-O-(p-Tosyloxy)-acetyl-13ß-chlormilbemycin D

Z35. 5-O-Chloracetyl-13ß-fluormilbemycin $A_3$

Z36. 5-O-Chloracetyl-13ß-fluormilbemycin $A_4$

Z37. 5-O-Chloracetyl-13-desoxy-13ß-fluor-22,23-dihydro-avermectin-Bla-aglykon

Z38. 5-O-Bromacetyl-13ß-fluormilbemycin $A_4$

Z39. 5-O-Bromacetyl-13ß-fluormilbemycin D

Z40. 5-O-Bromacetyl-13-desoxy-13ß-fluor-22,23-dihydro-avermectin-Bla-aglykon

Z41. 5-O-Fluoracetyl-13ß-fluormilbemycin $A_4$

Z42. 5-O-Fluoracetyl-13ß-fluormilbemycin D

Z43. 5-O-Jodacetyl-13ß-fluormilbemycin D

Z44. 5-O-Azidoacetyl-13ß-fluormilbemycin $A_4$

Z45. 5-O-Azidoacetyl-13ß-fluormilbemycin D

Z46. 5-O-Azidoacetyl-13-desoxy-13ß-fluor-22,23-dihydro-avermectin-Bla-aglykon

Z47. 5-O-Chloracetyl-13ß-brommilbemycin $A_3$

Z48. 5-O-Chloracetyl-13ß-brommilbemycin D

Z49. 5-O-Chloracetyl-13-desoxy-13ß-brom-22,23-dihydro-avermectin-B1a-aglykon

Z50. 5-O-Bromacetyl-13ß-brommilbemycin $A_4$

Z51. 5-O-Bromacetyl-13ß-brommilbemycin D

Z52. 5-O-Bromacetyl-13-desoxy-13ß-brom-22,23-dihydro-avermectin-B1a-aglykon

Z53. 5-O-Fluoracetyl-13ß-brommilbemycin $A_4$

Z54. 5-O-Fluoracetyl-13ß-brommilbemycin D

Z55. 5-O-Azidoacetyl-13ß-brommilbemycin D.

Herstellung von Verbindungen der Formel I

H-31. Herstellung von 5-O-(1,2,4-Triazol-1'-yl)-acetyl-milbemycin D (= Verb. Nr. 2.1)

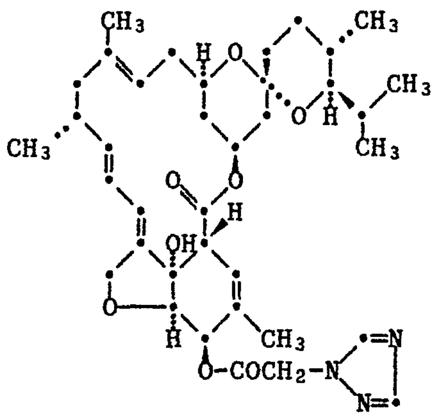

150 mg 5-O-Chloracetyl-milbemycin D und 33 mg 1,2,4-Triazol wurden in 5 ml trockenem Dimethylformamid gelöst und 40 Stunden bei einer Badtemperatur von 120°C unter Argonatmosphäre gerührt. Die dunkle Reaktionslösung wurde mit Eiswasser verdünnt und dreimal mit je 3 ml Ether extrahiert. Das so gewonnene dunkelbraune Harz wurde über eine Silicagelsäule mit Methylen/Methanol (95:5) als Laufmittel chromatographiert. Die Hauptfraktion ergab 70 mg einer gelblichen amorphen Masse.

$^1H$-NMR (300 MHz, $CDCl_3$, TMS) (s) ($-CH_2$-Triazol), 8,0 und 8,25 (2 s) (Triazol-Ringprotonen), 3,26 (schmales Multiplett) ($C_2$-H). Massenspektrum m/e: 665 ($M^+$, $C_{37}H_{51}N_3O_8$).

H-32. Herstellung von 5-O-(4'-Methyl-imidazol-1'-yl)-acetyl-milbemycin D und 5-O-(5'-Methylimidazol-1'-yl)-acetyl-milbemycin D (= Verb. Nr. 2.2 und 2.3)

150 mg 5-O-Chloracetyl-milbemycin D und 37,7 mg 4(5) Methylimidazol wurden 3 Stunden in 5 ml Dimethylformamid bei 100°C Badtemperatur unter Argon gerührt. Dünnschichtchromatographie zeigte die vollständige Umsetzung an. Das Reaktionsgemisch wurde auf Eiswasser gegossen und dreimal mit je 3 ml Ether extrahiert. Die Etherlösung wurde dreimal mit Wasser gewaschen und eingedampft. Der feste Rückstand wurde mittels Silicagel-Dickschichtchromatographie mit Methylenchlorid/Methanol (95:5) als Laufmittel in die 2 Isomeren getrennt. Man erhielt 35,8 mg Produkt vom Smp. 136-139°C; 26,6 mg Produkt vom Smp. 122-126°C;

$^1H$-NMR (250 MHz; $CDCl_3$, TMS): 4,85 (s) ($-CH_2$-Imidazol); 7,04; 7,12; 7,72; 8,02 (4 s) (Imidazol-Ringprotonen); 3,26 (schmales Multiplett) ($C_2$-H); Massenspektrum m/e: 678 ($M^+$, $C_{39}H_{54}N_2O_8$). Die Strukturzuordnung in 4-Methyl- und 5-Methyl-Derivat der beiden Isomeren kann nicht sicher getroffen werden.

H-33. Herstellung von 5-O-Imidazol-1'-yl-acetyl-milbemycin D (= Verb. Nr. 2.4)

In zu H-32 analoger Weise wurde durch Umsetzung von 5-O-Chloracetyl-milbemycin D mit Imidazol das 5-O-(Imidazol-1'-yl)-acetylmilbemycin mit dem Smp. 108-112°C gewonnen.
$^1H$-NMR (250 MHz; $CDCl_3$, TMS); 4,90 (s) $-CH_2$-Imidazol; 3,25 (m) ($C_2$-H).

0184989

H-34. Herstellung von 5-O-(1,2,4-Triazol-1'-yl)-acetyl-milbemycin $A_4$ (R = $C_2H_5$) (= Verb. Nr. 1.1)

Zu einer Lösung von 500 mg Milbemycin $A_4$ und 400 mg 1,2,4-Triazol-1-yl-essigsäure in 20 ml absolutem Tetrahydrofuran gab man 390 mg Dicyclohexylcarbodiimid und rührte 2 Stunden bei 20-25°C. Dann werden 5 ml Pyridin zugefügt, und die Mischung wurde noch 15 Stunden gerührt. Nun wurde die Reaktionsmischung in Eiswasser gegossen und mit 1N Salzsäure bis zur schwach sauren Reaktion versetzt. Man extrahierte das Reaktionsprodukt mit Ether, filtrierte vom Ungelösten, trocknete und evaporierte die Etherlösung. Man erhielt ein Rohprodukt, das durch Behandeln mit Hexan kristallisierte: 650 mg blass-gelbliche Kristalle vom Smp. 80-85°C.
$^1$H-NMR (250 MHz; $CDCl_3$, TMS): 5,05 (s) (-$CH_2$-Triazol); 8,0 und 8,25 (2 s) (Triazol-Ringprotonen); 3,25 (schmales Multiplett) $C_2$-H).

H-35: Herstellung von 5-O-(1.2.4-Triazol-1'-yl)-acetyl-milbemycin D-Kupfer(II)-Komplex ( = Verb. Nr. 2.1 g)

Zu einer Lösung von 100 mg 5-O-(1.2.4-Triazol-1'-yl)acetyl-milbemycin D in 0.75 ml absolut. Ethanol fügte man eine Lösung von 12.7 mg Kupfer-(II)-chlorid in 0.5 ml Ethanol. Die anfangs klare grüne Lösung schied nach kurzer Zeit blaugrüne Kristalle ab. Man isolierte 85 mg der Titelverbindung, Smp. 178°C (Zers.).

H-36. Herstellung von 5-O-(1,2,4-Triazol-1'-yl)-acetyl-13β-chlor-milbemycin D (=Verb. Nr. 2.10)

Analog Beispiel 34 erhielt man aus 150 mg 13β-Chlor-milbemycin D und 81 mg 1,2,4-Triazol-1-yl-essigsäure, 141 mg der Titelverbindung.

$^1$H-NMR (250 mg, $CDCl_3$):
3,07 (bd, J=9) ($C_{25}$H)
4,09 (d, J=10) ($C_{13}$H)
5,07 (s) ($CH_2$-Triazol)
7,98 und 8,24 (2s) (Triazol-Ringprotonen)

H-37. Herstellung von 5-O-(1,2,4-Triazol-1'-yl)-acetyl-13β-chlor-milbemycin D-Kupfer (II)-Komplex (=Verb. 2.2b)

Durch Umsetzung von 44 mg 5-O-(1,2,4-Triazol-1'-yl)-acetyl-13β-chlor-milbemycin D mit 75 mg Kupfer (II)-chlorid·$2H_2O$ erhielt man analog H-35 29 mg der Titelverbindung.

H-38. Herstellung von 5-O-(1,2,4-Triazol-1'-yl)-acetyl-13β-chlor-milbemycin D-Zink-(II)-Komplex (=Verb. 2.2a)

Zu einer Lösung von 37 mg 5-O-(1,2,4-Triazol-1'-yl)-acetyl-13β-chlor-milbemycin D in 0,50 ml Ethanol wurde eine Lösung von 5 mg Zink-(II)-chlorid in 0,25 ml Ethanol gegeben. Nach Zugabe von 0,50 ml Wasser begannen sich Kristalle abzuscheiden. Man isolierte 38 mg der Titelverbindung.

$^1$H-NMR (250 MHz, $CDCl_3$):
3,07 (bd, J=9) ($C_{25}H$)
4,09 (d, J=11) ($C_{13}H$)
5,10 (s) ($CH_2$-Triazol)
8,08 und 8,48 (2 bs) (Triazol-Ringprotonen)

H-39. Herstellung von 5-O-(1,2,4-Triazol-1'-yl)-acetyl-13β-chlor-milbemycin $A_4$ (=Verb. Nr. 1.9)

Aus 150 mg 13β-Chlor-milbemycin $A_4$ und 83 mg 1,2,4-Triazol-1-yl-essigsäure erhielt man analog Vorschrift H-34 159 mg der Titelverbindung

$^1$H-NMR (250 MHz, $CDCl_3$):
3,06 (dt, $J_d$=3, $J_t$=10 ($C_{25}H$)
4,09 (d, J=10) ($C_{13}H$)
5,07 (s) ($CH_2$-Triazol)
7,98 und 8,24 (2s) (Triazol-Ringprotonen)

H-40. Herstellung von 5-O-(1,2,4-Triazol-1'-yl)-acetyl-13β-chlor-milbemycin $A_4$-Eisen-(II )-Komplex (=Verb. 1.1.c)

Zu einer Lösung von 30 mg 5-O-(1,2,4-Triazol-1'-yl)-acetyl-13β-chlor-milbemycin $A_4$ in 3 ml Benzol/Methanol 2:1 wurden 0,50 ml einer Lösung von 174 mg Eisen-(II)-chlorid•$4H_2O$ in 10 ml Ethanol/Wasser 9:1 gegeben. Das Reaktionsgemisch wurde nach kurzem Stehenlassen eingedampft, mit wenig Benzol versetzt und gefriergetrocknet. Man erhielt 37 mg der Titelverbindung als gelbbraunes Pulver.

H-41. Herstellung von 5-O-(1,2,4-Triazol-1'-yl)-acetyl-13β-chlor-milbemycin $A_4$-Kupfer-(II)-Komplex (=Verb. 1.1d)

Zu einer Lösung von 40 mg 5-O-(1,2,4-Triazol-1'-yl)-acetyl-13β-chlor-milbemycin $A_4$ in 0,50 ml Ethanol wurden 0,50 ml einer Lösung von 86 mg wasserfreiem Kupfer-(II)-chlorid in 10 ml Ethanol gegeben. Nach kurzem Stehenlassen wurde das Reaktionsgemisch eingedampft, dann mit 5 ml Benzol versetzt und gefriergetrocknet. Man erhielt 44 mg der Titelverbindung als grünliches Pulver.

H-42. Herstellung von 5-O-(1,2,4-Triazol-1'-yl)-acetyl-13β-chlor-milbemycin $A_4$-Zink-(II) -Komplex (=Verb. 1.1a)

Zu einer Lösung von 39 mg 5-O-(1,2,4-Triazol-1'-yl)-acetyl-13β-chlor-milbemycin $A_4$ in 0,50 ml Ethanol wurden 0,50 ml einer Lösung von 86 mg Zink-(II)-chlorid in 10 ml Ethanol gegeben. Das Reaktionsgemisch wurde dann eingedampft, mit 3 ml Benzol versetzt und gefriergetrocknet. Man erhielt 43 mg der Titelverbindung als farbloses Pulver.

$^1$H-NMR (250 , $CDCl_3$):
3,07 (dt, $J_d$=3, $J_t$=10) ($C_{25}H$)
4,09 (d, J=10) ($C_{13}H$)
5,19 (6S) ($CH_2$-Triazol)
8,28 und 9,02 (2bs) (Triazol-Ringprotonen)

Nach Art der obigen Beispiele oder nach einer der eingangs beschriebenen Methoden lassen sich weitere erfindungsgemässe Wirkstoffe herstellen:

Milbemycin $A_4$-Reihe:

| Nr. | |
|---|---|
| 1.2 | 5-O-(Imidazol-1'-yl)acetyl-, |
| 1.3 | 5-O-(Pyrazol-1'-yl)acetyl-, |
| 1.4 | 5-O-(2'-Methylimidazol-1'-yl)acetyl-, |
| 1.5 | 5-O-(Tetrazol-1'-yl)acetyl-, |
| 1.6 | 5-O-(Imidazol-1'-yl)acetyl-13ß-fluor-, |
| 1.7 | 5-O-(Imidazol-1'-yl)acetyl-13ß-chlor-, |
| 1.8 | 5-O-(2'-Ethyl-imidazol-1'-yl)acetyl-13ß-chlor-, |
| 1.9 | 5-O-(1,2,4-Triazol-1'-yl)acetyl-13ß-chlor-, |
| 1.10 | 5-O-(Pyrazol-1'-yl)acetyl-13ß-chlor-, |
| 1.11 | 5-O-(1,2,4-Triazol-1'-yl)acetyl-13ß-fluor-, |
| 1.12 | 5-O-(4'(5')Methyl-imidazol-1'-yl)acetyl-13ß-brom-, |
| 1.13 | 5-O-(1,2,4-Triazol-4'-yl)acetyl-13ß-chlor-, |
| 1.14 | 5-O-(Imidazol-1'-yl)acetyl-13ß-jod-, |
| 1.15 | 5-O-(1,2,3-Triazol-1'-yl)acetyl-13ß-chlor-, |
| 1.16 | 5-O-(1,2,4-Triazol-1'-yl)acetyl-13ß-brom-, |
| 1.17 | 5-O-(1,2,4-Triazol-1'-yl)acetyl-13ß-jod-, |
| 1.18 | 5-O-(Pyrazol-1'-yl)acetyl-13ß-jod-. |

Milbemycin D-Reihe:

| Nr. | |
|---|---|
| 2.5 | 5-O-(2'-Methylimidazol-1'-yl)acetyl-, |
| 2.6 | 5-O-(Tetrazol-1'-yl)acetyl-, |
| 2.7 | 5-O-(Pyrazol-1'-yl)acetyl-, |
| 2.8 | 5-O-(1,2,3-Triazol-1'-yl)acetyl-, |
| 2.9 | 5-O-(1,2,4-Triazol-1'-yl)acetyl-13ß-fluor-, |
| 2.10 | 5-O-(1,2,4-Triazol-1'-yl)acetyl-13ß-chlor-, |
| 2.11 | 5-O-(Imidazol-1'-yl)acetyl-13ß-chlor-, |
| 2.12 | 5-O-(2'-Ethylimidazol-1'-yl)acetyl-13ß-chlor-, |
| 2.13 | 5-O-(Pyrazol-1'-yl)acetyl-13ß-fluor-, |

2.14 5-O-(Pyrazol-1'-yl)acetyl-13β-chlor-,

2.15 5-O-[4'(5')-Methylimidazol-1'-yl]-13β-chlor-,

2.16 5-O-(Tetrazol-1'-yl)acetyl-13β-fluor-,

2.17 5-O-(1,2,4-Triazol-4'-yl)acetyl-13β-fluor-,

2.18 5-O-(1,2,4-Triazol-4'-yl)acetyl-13β-chlor-,

2.19 5-O-(1,2,3-Triazol-1'-yl)acetyl-13β-fluor-,

2.20 5-O-(1,2,3-Triazol-1'-yl)acetyl-13β-chlor-,

2.21 5-O-(Imidazol-1'-yl)acetyl-13β-fluor-,

2.22 5-O-(1,2,4-Triazol-1'-yl)acetyl-13β-brom-,

2.23 5-O-(1,2,4-Triazol-1'-yl)acetyl-13β-jod-

2.24 5-O-(2-Ethyl-4-methyl-imidazol-1'-yl)acetyl-.

Milbemycin $A_3$-Reihe:

3.1 5-O-(Pyrazol-1'-yl)acetyl-,

3.2 5-O-(1,2,4-Triazol-1'-yl)acetyl-,

3.3 5-O-(1,2,4-Triazol-4'-yl)acetyl-,

3.4 5-O-(Imidazol-1'yl)acetyl-,

3.5 5-O-(Pyrazol-1'-yl)acetyl-13β-chlor-,

3.6 5-O-(1,2,4-Triazol-1'-yl)acetyl-13β-chlor-,

3.7 5-O-(Imidazol-1'-yl)acetyl-13β-chlor-.

13-Desoxi-22,23-dihydro-avermectin-Bla-aglykon-Reihe:

4.1 5-O-(Pyrazol-1'-yl)acetyl-,

4.2 5-O-(1,2,3-Triazol-1'-yl)acetyl-,

4.3 5-O-(1,2,4-Triazol-1'-yl)acetyl-,

4.4 5-O-(Tetrazol-1'-yl)acetyl-,

4.5 5-O-[4'(5')Isopropylimidazol-1'-yl)acetyl-,

4.6 5-O-(Imidazol-1'-yl)acetyl-,

4.7 5-O-(Pyrazol-1'-yl)acetyl-13β-chlor-,

4.8 5-O-(1,2,3-Triazol-1'-yl)acetyl-13β-chlor-,

4.9 5-O-(1,2,4-Triazol-1'-yl)acetyl-13β-chlor-,

4.10 5-O-(Tetrazol-1'-yl)acetyl-13β-chlor-,

4.11 5-O-(Imidazol-1'-yl)acetyl-13β-chlor-,

4.12 5-O-(1,2,4-Triazol-1'-yl)acetyl-13β-fluor-,

4.13 5-O-(2'-Imidazol-1'-yl)acetyl-13β-chlor-.

Analog dem Beispiel H-9 lassen sich von den vorgenannten Verbindungen Metallkomplexe herstellen, z.B.

Komplex-Salze von 5-O-(1,2,4-Triazol-1'-yl)acetyl-milbemycin D

| | | | |
|---|---|---|---|
| 2.1a | Zink-Komplex | (• 1/2 $ZnCl_2$) | Smp. 183°C (Zers.) |
| 2.1b | Mangan-Komplex | (• 1/2 $MnCl_2$) | |
| 2.1c | Nickel-Komplex | (• 1/2 $NiCl_2$) | |
| 2.1d | Kobalt-Komplex | (• 1/2 $CoCl_2$) | |
| 2.1e | Cadmium-Komplex | (• 1/2 $CdSO_4$) | |
| 2.1f | Eisen-Komplex | (• 1/2 $FeSO_4$) | |
| 2.1g | Kupferkomplex | (• 1/2 $CuCl_2$) | Smp. 178°C (Zers.) |

Komplex-Salze von 5-O-(1,2,4-Triazol-1'-yl)-13β-chlor-milbemycin D

| | | | |
|---|---|---|---|
| 2.2a | Zink-Komplex | (• 1/2 $ZnCl_2$) | Smp. 182°C (Zers.) |
| 2.2b | Kupfer-Komplex | (• 1/2 $CuCl_2$) | Smp. 174°C (Zers.) |

Komplex-Salze von 5-O-(1,2,4-Triazol-1'-yl)acetyl-13β-chlor-milbemycin $A_4$

| | | | |
|---|---|---|---|
| 1.1a | Zink-Komplex | (• 1/2 $ZnCl_2$) | Smp. 176°C (Zers.) |
| 1.1b | Mangan-Komplex | [• 1/2 $Mn(NO_3)_2$] | Smp. 183°C (Zers.) |
| 1.1c | Eisen-Komplex | (• 1/2 $FeCl_2$) | Smp. 181°C (Zers.) |
| 1.1d | Kupfer-Komplex | (• 1/2 $CuCl_2$) | Smp. 179°C (Zers.) |

Die vorstehend genannten Milbemycin-Derivate der Formel I können über ihre Salze (durch Behandeln mit der entsprechenden Säure) gereinigt und auch in dieser Form in den Handel gebracht oder angewendet werden. Geeignete Salze sind z.B. die Hydrochloride, Sulfate, Nitrate, Tosylate, Tartrate oder Oxalate.

0184989

Formulierungsbeispiele für den Wirkstoff der Formel I

(% = Gewichtsprozent)

| Spritzpulver | a) | b) | c) |
|---|---|---|---|
| Wirkstoff Nr. Z1-Z55 oder 1.1-4.13 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff Nr. Z1-Z55 oder 1.1-4.13 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff Nr. Z1-Z55 oder 1.1-4.13 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

<u>Extruder Granulat</u>

| | |
|---|---|
| Wirkstoff Nr. Z1-Z55 oder 1.1-4.13 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

<u>Tabellen bzw. Boli</u>

| | | |
|---|---|---|
| I | Wirkstoff Nr. Z1-Z55 oder 1.1-4.13 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen.

III Phasen I und II mischen und zu Tabletten oder Boli verpressen.

Falls die Verbindungen der Formel I oder V bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I oder V bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Verbindungen der Formeln I und V z.B. Nr. Z1, Z8, 1.1, 2.1g, 2.1a oder 1.1c erzielten bereits bei einer Wirkstoffkonzentration von 3 ppm eine vollständige Abtötung nach 24 Stunden.

B-2. Wirkung gegen pflanzenschädigende Akariden

OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25°C.

Nach sieben Tagen wird unter dem Binokular auf Prozentsatz mobiler Stadien (Adulte und Nymphen) und auf vorhandene Eier ausgewertet.

Die Verbindungen der Formel I und V z.B. Z1, Z3, Z7, Z8 oder Z10 erzielten bereits bei einer Wirkstoffkonzentration von 0,4 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Alle mit physikalischen Daten genannten Verbindungen der Formel I und V erzielten mit 250 ppm eine Wirkung von 100 %.

B-4. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 5, 0,5 oder 0,05 µg pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I und V wie z.B. die der Nr. Z1, Z3, Z7, Z8, Z10, Nr. 2.1, 2.2 bis 2.3, 2.4, 2.1g, 2.1a, 1.9, 2.10, 1.1a, 1.1c oder 1.1d erzielten eine $IR_{90}$ von 0,5 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer derjenigen der Verbindungen der Formeln I oder V, die mit physikalischen Daten genannt sind, bei 1 mg/kg behandelt wurden, zeigten im Vergleich zu unbehandelten, aber infizierten Vergleichsgruppen keinen Nematodenbefall (= komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Die Verbindungen der Formel I und z.B. Nr. Z1, Z7, Z8, 1.1 oder 1c erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I und V wie z.B. Nr. Z1, 2.1, 1.1, 2.1a oder 1.1d bewirkten in diesem Test bei einer Konzentration von 1,6 ppm bereits nach einem Tag eine vollständige Abtötung sämtlicher Larven.

<u>Patentansprüche</u> für die Vertragsstaaten: DE, GB, FR, CH, LI, IT, NL, BE, SE, LU

1. Milbemycin-Derivat der Formel I

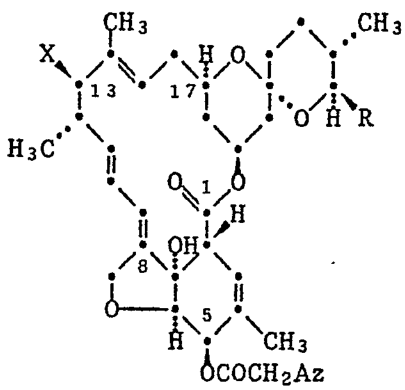

(I)

worin X Wasserstoff oder ß-Halogen bedeutet, R Methyl, Ethyl, Isopropyl oder sek.Butyl ist und Az einen in 1-Position verknüpften 5-gliedrigen heterocyclisch aromatischen Ring mit zwei bis vier Stickstoffatomen darstellt, der unsubstituiert oder ein- bis zweifach durch $C_1$-$C_6$ Alkyl substituiert ist, unter Einschluss seiner Säureadditionssalze und Metallkomplexverbindungen.

2. Eine Verbindung der Formel I gemäss Anspruch 1, worin R für Isopropyl steht und X und Az wie in Anspruch 1 definiert sind.

3. Eine Verbindung der Formel I gemäss Anspruch 1, worin Az in 1-Position verknüpftes Imidazol, Pyrazol, 1,2,3-Triazol, 1,2,4-Triazol, 1,3,4-Triazol, Tetrazol, 2-Ethyl-4-methylimidazol, 2-Isopropylimidazol, Methylimidazol, 3,5-Dimethyltriazol, Ethyltriazol oder 3,4-Diethylpyrazol bedeutet.

4. Eine Verbindung der Formel I gemäss Anspruch 3, worin X Wasserstoff oder ß-Chlor, R Ethyl oder Isopropyl und "Az" 1,2,4-Triazol-1-yl bedeuten.

5. 5-O-(1,2,4-Triazol-1'-yl)acetyl-13-β-chlor-milbemycin-D oder eines seiner Salze oder Metallkomplexe.

6. Ein Metallkomplex gemäss Anspruch 5 mit einem der Metalle Kupfer, Zink, Mangan, Chrom, Eisen, Nickel, Kobalt oder Molybdän.

7. Verfahren zur Herstellung einer Verbindung der Formel I gemäss Anspruch 1, dadurch gekennzeichnet, dass man wahlweise eine Verbindung der Formel II

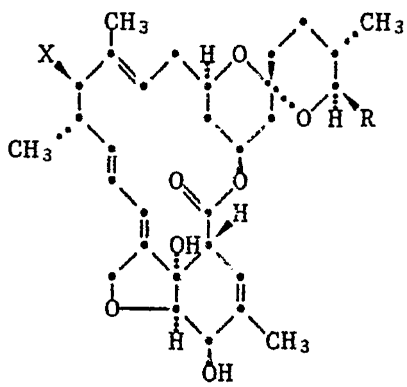

(II)

worin R und X die für Verbindung I aufgeführten Bedeutungen haben, mit einer 2-Azolylessigsäure oder einem ihrer Säurederivate der Formel

$$Az - CH_2 - COZ$$

zur Reaktion bringt, wobei Z Halogen, -OH oder die halbe Sauerstoff-Funktion im Säureanhydrid bedeutet, oder dass man in zwei Teilschritten die Verbindung der Formel II mit einer substituierten Essigsäure der Formel

$$Y - CH_2 - COZ ,$$

worin Y für Halogen, Azido oder eine andere nucleophil leicht ersetzbare Abgangsgruppe bedeutet und Z der oben genannten Definition gehorcht, zu einer Verbindung der Formel V umsetzt

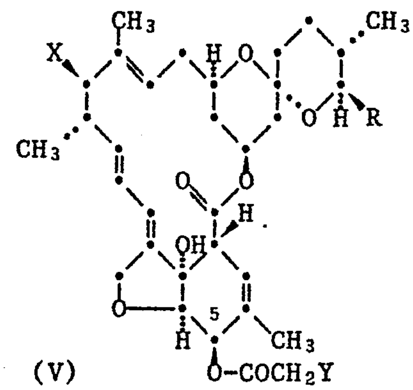

(V)

und diese mit einem Azol "Az" reagieren lässt.

8. Milbemycin-Derivat der Formel V

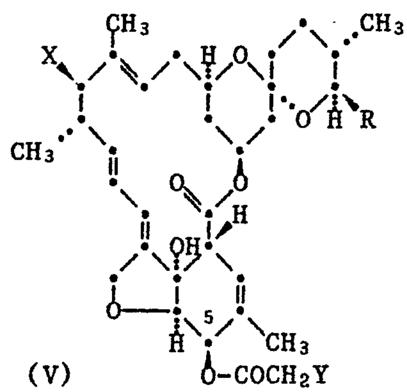

(V)

worin X Wasserstoff oder ß-Halogen bedeutet, R Methyl, Ethyl, Isopropyl oder sek.Butyl ist, und Y für Halogen, Azido, Mesyl oder Tosyl steht.

9. Schädlingsbekämpfungsmittel enthaltend als mindestens einen Wirkstoff eine Verbindung der Formel I gemäss Anspruch 1 oder eines ihrer Säureadditionssalze oder Metallkomplexverbindungen oder eine Verbindung der Formel V gemäss Anspruch 5, zusammen mit einem geeigneten Trägermaterial.

10. Mittel gemäss Anspruch 9 enthaltend eine Verbindung der Formel I gemäss einem der Ansprüche 2 bis 6.

11. Verwendung der Verbindungen der Formel I gemäss Anspruch 1 oder der Formel V gemäss Anspruch 8 zur Bekämpfung von Insekten und Schädlingen der Ordnung Acarina.

12. Verwendung der Verbindung der Formel I gemäss Anspruch 1 oder der Formel V gemäss Anspruch 8 zur Bekämpfung von Nematoden.

13. Verwendung der Verbindung der Formel I gemäss Anspruch 1 oder der Formel V gemäss Anspruch 8 zur Bekämpfung von Ekto- und Endoparasiten am tierischen Warmblüter.

14. Verfahren zur Bekämpfung von tier- oder pflanzenparasitären Schädlingen, dadurch gekennzeichnet, dass man die Verbindung der Formel I gemäss Anspruch 1 oder die Verbindung der Formel V gemäss Anspruch 8 auf oder in die Tiere bzw. Pflanzen appliziert.

FO 7.5/HL/sch*/cw*

Patentansprüche für den Vertragsstaat Oesterreich

1. Schädlingsbekämpfungsmittel enthaltend neben üblichen Formulierungshilfsstoffen ein Milbemycin-Derivat der Formel I

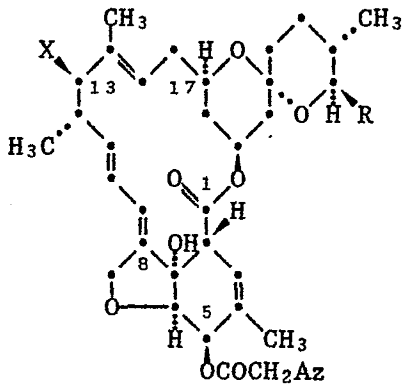

(I)

worin X Wasserstoff oder ß-Halogen bedeutet, R Methyl, Ethyl, Isopropyl oder sek.Butyl ist und Az einen in 1-Position verknüpften 5-gliedrigen heterocyclisch aromatischen Ring mit zwei bis vier Stickstoffatomen darstellt, der unsubstituiert oder ein- bis zweifach durch $C_1$-$C_6$ Alkyl substituiert ist, unter Einschluss seiner Säureadditionssalze und Metallkomplexverbindungen.

2. Mittel nach Anspruch 1 enthaltend eine Verbindung der Formel I, worin R für Isopropyl steht und X und Az wie in Anspruch 1 definiert sind.

3. Mittel nach Anspruch 1 enthaltend eine Verbindung der Formel I, worin Az in 1-Position verknüpftes Imidazol, Pyrazol, 1,2,3-Triazol, 1,2,4-Triazol, 1,3,4-Triazol, Tetrazol, 2-Ethyl-4-methylimidazol, 2-Isopropylimidazol, Methylimidazol, 3,5-Dimethyltriazol, Ethyltriazol oder 3,4-Diethylpyrazol bedeutet.

4. Mittel nach Anspruch 3 enthaltend eine Verbindung der Formel I, worin X Wasserstoff oder ß-Chlor, R Ethyl oder Isopropyl und "Az" 1,2,4-Triazol-1-yl bedeuten.

5. Mittel nach Anspruch 1 enthaltend als Wirkstoff 5-O-(1,2,4-Triazol-1'-yl)acetyl-13-β-chlor-milbemycin-D oder eines seiner Salze oder Metallkomplexe.

6. Mittel nach Anspruch 5 enthaltend einen Metallkomplex mit einem der Metalle Kupfer, Zink, Mangan, Chrom, Eisen, Nickel, Kobalt oder Molybdän.

7. Verfahren zur Herstellung einer Verbindung der Formel I gemäss Anspruch 1, dadurch gekennzeichnet, dass man wahlweise eine Verbindung der Formel II

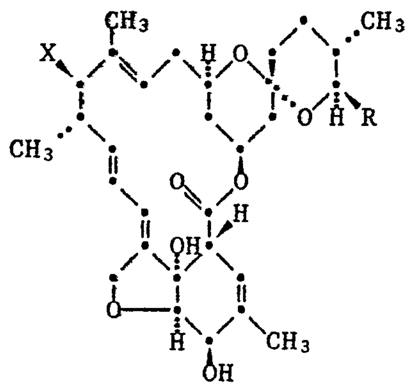

(II)

worin R und X die für Verbindung I aufgeführten Bedeutungen haben, mit einer 2-Azolylessigsäure oder einem ihrer Säurederivate der Formel

$$Az - CH_2 - COZ$$

zur Reaktion bringt, wobei Z Halogen, -OH oder die halbe Sauerstoff-Funktion im Säureanhydrid bedeutet, oder dass man in zwei Teilschritten die Verbindung der Formel II mit einer substituierten Essigsäure der Formel

$$Y - CH_2 - COZ ,$$

worin Y für Halogen, Azido oder eine andere nucleophil leicht ersetzbare Abgangsgruppe bedeutet und Z der oben genannten Definition gehorcht, zu einer Verbindung der Formel V umsetzt

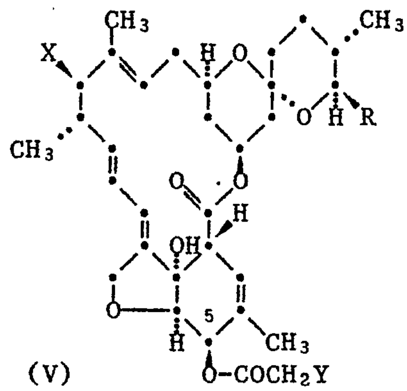

(V)

und diese mit einem Azol "Az" reagieren lässt.

8. Schädlingsbekämpfungsmittel enthaltend neben üblichen Formulierungshilfstoffen ein Milbemycin-Derivat der Formel V

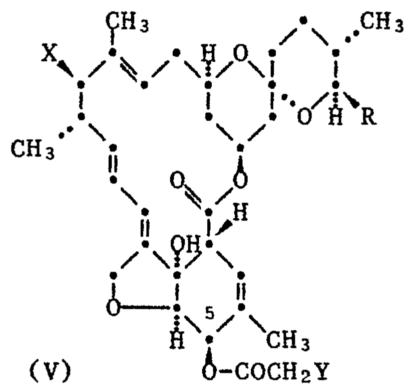

(V)

worin X Wasserstoff oder ß-Halogen bedeutet, R Methyl, Ethyl, Isopropyl oder sek.Butyl ist, und Y für Halogen, Azido, Mesyl oder Tosyl steht.

9. Verwendung der Verbindungen der Formel I gemäss Anspruch 1 oder der Formel V gemäss Anspruch 8 zur Bekämpfung von Insekten und Schädlingen der Ordnung Acarina.

10. Verwendung der Verbindung der Formel I gemäss Anspruch 1 oder der Formel V gemäss Anspruch 8 zur Bekämpfung von Nematoden.

11. Verwendung der Verbindung der Formel I gemäss Anspruch 1 oder der Formel V gemäss Anspruch 8 zur Bekämpfung von Ekto- und Endoparasiten am tierischen Warmblüter.

12. Verfahren zur Bekämpfung von tier- oder pflanzenparasitären Schädlingen, dadurch gekennzeichnet, dass man die Verbindung der Formel I gemäss Anspruch 1 oder die Verbindung der Formel V gemäss Anspruch 8 auf oder in die Tiere bzw. Pflanzen appliziert.

FO 7.5/HL/sch*/cw*/sm*

Europäisches Patentamt

**EUROPÄISCHER RECHERCHENBERICHT**

0184989

Nummer der Anmeldung

EP 85 81 0554

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 101, Nr. 3, 16. Juli 1984, Seite 578, Zusammenfassung Nr. 23232v, Columbus, Ohio, US; & JP - A - 59 16 894 (SANKYO CO., LTD.) 28.01.1984<br>* Zusammenfassung *<br>----- | 1-14 | C 07 D 493/22<br>A 61 K 31/35<br>A 01 N 43/24 //<br>(C 07 D 493/22<br>C 07 D 313:00<br>C 07 D 311:00<br>C 07 D 311:00<br>C 07 D 307:00 ) |
| | | | **RECHERCHIERTE SACHGEBIETE (Int. Cl.4)** |
| | | | C 07 D 493/00<br>A 61 K 31/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 24-02-1986 | VERHULST W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82